# EUROPÄISCHE PATENTSCHRIFT

(11) **EP 1 844 323 B1**
(45) Veröffentlichungstag und Bekanntmachung des Hinweises auf die Patenterteilung: **17.09.2008**
(21) Anmeldenummer: 05798678.8
(22) Anmeldetag: 09.11.2005
(51) Int. Cl.: G01N 25/56, A01G 25/16

(54) **VORRICHTUNG UND VERFAHREN ZUR BESTIMMUNG VON FEUCHTIGKEIT IN EINEM MEDIUM**
DEVICE AND METHOD FOR DETERMINING THE MOISTURE CONTENT IN A MEDIUM
DISPOSITIF ET PROCEDE DE DETERMINATION DE L'HUMIDITE DANS UN MILIEU

(30) Priorität: 02.02.2005 CH 149052005; 07.06.2005 CH 960052005
(43) Veröffentlichungstag der Anmeldung: 17.10.2007
(73) Patentinhaber: Plantcare AG, 8332 Russikon (CH)
(72) Erfinder: SCHMIDT, Walter, CH-8332 Russikon (CH)
(74) Vertreter: Frei Patent Attorneys
(86) Internationale Anmeldenummer: PCT/CH2005/000663
(87) Internationale Veröffentlichungsnummer: WO 2006/081693

(56) Entgegenhaltungen:
- EP-A- 1 308 085
- WO-A-98/52027
- DE-A1- 3 510 379
- DE-A1- 4 340 775
- DE-A1- 10 164 018

## Beschreibung

Die Erfindung bezieht sich auf Feuchtigkeitssensoren und ein Verfahren zur Messung von Feuchtigkeit in einem Medium, insbesondere von Erden.

Während die Messung der relativen Luftfeuchtigkeit sowohl über konventionelle, als auch mittels elektronischer Sensoren heute einfach möglich ist, ist die Messung der Feuchtigkeit von Gemengen, insbesondere von Böden bzw. Pflanzensubstraten, nach wie vor problematisch.

Elektronisch arbeitende Feuchtesensoren arbeiten meist auf der Basis von Feuchte absorbierenden dielektrischen Schichten, die eine vom Wassergehalt stark abhängige Dielektrizitätskonstante aufweisen. Diese Schichten werden zwischen zwei Elektrodenschichten eingebracht, die einen Plattenkondensator bilden und die Veränderung der Kapazität dieses Kondensators wird mittels einer angelegten Wechselspannung gemessen.

Dieses Prinzip ist zur Messung der Luftfeuchtigkeit bestens geeignet, zur Bestimmung der Bodenfeuchtigkeit jedoch nicht anwendbar. Einerseits müsste der Sensor in den Boden versenkt werden, wobei für eine zuverlässige Bestimmung der Feuchte eine direkte gasdurchlässige Verbindung zwischen dem Boden und dem Sensor, sowie auch eine gute Durchlüftung des Sensors gewährleistet sein muss. Eine direkte gasdurchlässige Verbindung bietet aber enorme Schwierigkeiten. Da der Boden nicht nur feucht, sondern auch nass sein kann, kommt die meist als poröses Filter konzipierte Gasverbindung direkt mit der Flüssigkeit in Berührung und wird verstopft. Der Filter wird auch durch Bodenbakterien besiedelt und es können Ionen durch den Filter in den Sensorbereich einwandern und diesen zerstören. Zudem ist der Sensor, einschliesslich der Ansteuerung und Auswertelektronik, teuer und daher für Massenanwendungen nicht geeignet.

Andere Methoden verwenden als Messgrösse die Veränderung des elektrischen Widerstandes mit zunehmender Feuchte eines geeigneten, Wasser aufsaugenden Materials, wie Gips oder Nylon, das mit der umgebenden Erde in Kontakt gebracht wird, Mikrowellen-Strahlung bestimmter Frequenz, die lokal in den Boden eingestrahlt wird oder kapazitive Sensoren, die über ein elektrisches Wechselfeld die Dielektrizitätskonstante der umgebenden Erde bestimmen. Alle diese Methoden sind nebst weiteren Nachteilen mit hohen Kosten und teilweise auch mit hohem technischem Aufwand verbunden.

Daher werden heute für Messungen an Bodensubstraten überwiegend Tensiometer - Systeme verwendet. Eine mit Wasser gesättigte poröse Tonzelle ist mittels Wasser gefülltem Plexiglasrohr luftdicht an ein Manometer zur Messung von Unterdruck angeschlossen. Durch Kontakt der Zelle mit dem Boden wird der vorhandene Unterdruck des Bodenwassers über die Tonzelle und die Wasserfüllung auf das Manometer übertragen und ist dort ablesbar. Bei abnehmender Bodenfeuchte steigt die Saugspannung des Bodenwassers, die als Unterdruck durch das Manometer angezeigt wird.

Dieses Prinzip wird heute insbesondere im Bereich der Bodenfeuchtemessung in Gärtnereien etc. aber auch bei Bewässerungssystemen für Balkone- und Terrassenpflanzen angewendet, mit folgenden Nachteilen. Der poröse Messkörper muss ganzflächig im direkten Kontakt mit dem umgebenen Substrat sein und kann daher leicht verstopfen, bzw. bei kalkhaltigem Wasser auch verkalken. Er muss daher immer wieder kontrolliert bzw. ausgewechselt werden. Der der dazu notwendige Wasservorrat muss kontrolliert und regelmässig nachgefüllt werden. Zudem sind die Herstellkosten eines derartigen Sensors relativ hoch und liegen im Bereich von 30 - 50 €. Eine direkte elektronische Auswertung ist mit beträchtlichem Aufwand verbunden, da dazu die Durchbiegung der Membrane in ein elektronisches Signal umgewandelt werden muss.

Neben Sensoren, die die Neutronen-Rückstreuung und Absorption als Messprinzip nutzen, gibt es noch eine Reihe weiterer Sensorprinzipien. Darunter findet sich auch das Prinzip der Messung der Wärmeleitfähigkeit des Bodens. Diese Methode hat sich aber, trotz verschiedener Anläufe bisher nicht durchgesetzt. So beschreibt WO9013812 ein Verfahren, bei dem sich Erde im Inneren eines Zylinders, welcher mit einem Heizelement und einem Temperatursensor versehen ist, befindet. Das Heizelement erzeugt einen Wärmepuls, dessen Aufheizeffekt auf die Erde mit dem Temperatursensor gemessen und als Mass für die Feuchte gewertet wird. Insbesondere die Wärmeleitung wird stark von der Art und Grösse der nicht klar definierbaren Berührungsfläche zwischen Erde und Sensor bestimmt. Auch spielt die Art und Porosität der Erde eine grosse Rolle.

Ein weiteres Verfahren mit Messung von Wärmeleitfähigkeit wird in JP03061845A2 beschrieben. Dabei sind entlang eines Messstabes ein Heizelement und ein Temperaturfühler in einem gewissen Abstand angeordnet. Das Heizelement heizt die umgebende Erde auf und transportiert einen Teil der Wärmeenergie über die umgebende Erde an den Temperaturfühler, der eine Temperaturerhöhung anzeigt. Daraus kann auf den Wassergehalt der Umgebung geschlossen werden. Auch dieser Sensor ist unzuverlässig, da die Grenzfläche zwischen Heizelement und Erde, wie auch zwischen Temperaturfühler und Erde nicht definiert ist.

Um den Nachteil der nicht klar definierbaren Berührungsfläche zwischen Erde und Sensor zu umgehen, wurde in DE2536777 ein Verfahren entwickelt, bei dem der Temperaturfühler mit einer künstlichen Standarderde umgeben wird, sodass die Grenzfläche vom Fühler zur Umgebung besser definiert ist. Dabei wird die Temperaturerhöhung vor und nach einem kurzen Heizimpuls gemessen und die Differenz zur Bestimmung des Feuchtegehaltes des Bodens herangezogen. Es ist auch bekannt, dass die Standarderde durch eine poröse Keramik ersetzt wurde.

Dieses Verfahren hat den Nachteil, dass die Differenz zwischen der Start- und Endtemperatur nur sehr wenig vom Wassergehalt abhängt, d.h. die Sensitivität eines solchen Verfahrens ist sehr schlecht. Zudem weist die Standarderde selbst eine hohe Wärmekapazität und Wärmeleitfähigkeit auf, welche die Messung beeinflusst.

In der Schrift WO 98/52027 wird ein kostengünstiger Silizium-Chip beschrieben, welcher auch als Feuchtesensor verwendet wird. Darin ist die Oberfläche einer Silizium-Membran mit einem saugfähigen Material bedeckt ist, um dem Sensor Feuchtigkeit aus der Umgebung zuzuführen. Die Messflächen sind sehr klein, so dass ein Übergangswiderstand Sensor-Erde, bei einem heterogenen Erdgemisch völlig zufällig ist. Zudem dominiert bei der Messung die Wärmeleitfähigkeit der Erde durch das dünne Material hindurch, so dass die Messung durch eine unbestimmte (thermische) Zusammensetzung der umgebenden Erde unzuverlässig ist.

In der Schrift DE 43 40 775 wird ebenfalls erkannt, dass ein guter Kontakt zwischen Sensorgehäuse und Erde wichtig ist. Dabei wird vorgeschlagen, die Gehäusewände mittels Druck ans Erdreich anzupressen. Dabei wird jedoch das Substrat lokal verdichtet und seine thermischen Eigenschaften ändern sich. Zudem ist Erdreich oft kompressibel und weicht einem Druck aus. Trocknet das Substrat anschliessend, kann sich der Sensor vom Substrat lösen, was einen stark veränderten Wärmewiderstand an der Grenzfläche nach sich zieht.

Andere Messverfahren, wie bspw. in US 5,287,734 beschrieben, verwenden eine poröse Keramik, die zwischen einem Heizelement und einem Temperaturfühler angeordnet wird. Saugt sich die Keramik mit Wasser der umgebenden Erde voll, verändert sich der Wärmeleitwert der Keramik, was auf den Wassergehalt der Erde rückschliessen lässt. Der Nachteil dieser Verfahren liegt darin, dass sich die Keramik nie im gleichen Masse mit Wasser anreichert, wie die umgebende Erde, da sich die Verteilung der wassersaugenden Poren zwischen Erde und Keramik stark unterscheidet. Ausserdem bilden sich Lufteinschlüsse, da die Luft aus dem Keramikkörper nicht mehr entweichen kann. Da nur ein Bruchteil des Volumens der Keramik in Form von Poren vorliegt, ist die Sensitivität schlecht. Bedingt durch die relativ hohe Wärmeleitfähigkeit der Keramik wirkt sich die Wärmeleitfähigkeit des umgebenden Bodens stark auf die Messung aus.

Aus den oben erwähnten Gründen und insbesondere wegen der hohen Kosten haben sich bisher keine Verfahren durchgesetzt, die auf dem thermischen Messprinzip beruhen.

Aufgabe der Erfindung ist es, eine Vorrichtung für ein thermisches Messverfahren, zu schaffen, welche oben genannte technische Nachteile vermeidet und welche insbesondere kostengünstig hergestellt werden kann, zuverlässig und sensitiv in Bezug auf den Wassergehalt des umgebenden Mediums ist.

Die Aufgabe wird durch die Vorrichtung gelöst, wie sie in den Patentansprüchen definiert ist.

Die Erfindung basiert auf dem Messprinzip mit einer dynamischen Temperaturmessung. In der Spitze eines Messfühlers ist beispielsweise ein von der Temperatur abhängiger elektrischer Widerstand eingebaut, der kurzzeitig auf eine höhere Temperatur aufgeheizt wird. Die Heizung kann durch ein zusätzliches Heizelement oder auch direkt durch einen Messwiderstand erfolgen.

Bei einer gegebenen Heizleistung erwärmt sich die Messspitze ausgehend von der Umgebungstemperatur, wobei die Temperaturerhöhung im allgemeinen von der Wärmekapazität des Sensors selbst, der Wärmeleitfähigkeit eines den Sensor umhüllenden Materials, sowie auch von der Wärmekapazität und Wärmeleitfähigkeit des umgebenden Mediums abhängig ist. Letztere sind wiederum abhängig von der Zusammensetzung des umgebenden Mediums, insbesondere von dessen Wassergehalt. Bei günstiger Auslegung des Sensors wird der überwiegende Teil der eingebrachten Wärmeenergie vorerst in der Sensorspitze gespeichert. Wird die Heizung nach einer gegebenen Zeit ausgeschaltet, so kühlt die Messspitze ab, wobei die Abkühlungskurve eine Aussage über die Zusammensetzung der Umgebung, insbesondere über deren Wassergehalt zulässt. Andererseits kann auch die Aufheizkurve eine Aussage über den Feuchtegehalt des umgebenden Materials zulassen.

Ist der Wassergehalt hoch, so wird sich die Messspitze beim Aufheizen langsamer erwärmen und nach dem Abschalten der Heizung sehr rasch wieder auf die Umgebungstemperatur abkühlen. Ist der Wassergehalt niedrig, so wird die Messspitze sich schneller aufheizen und langsamer abkühlen.

Zur elektronischen Auswertung können prinzipiell unterschiedliche Messgrössen, bzw. auch Kombinationen solcher herangezogen werden. Grundsätzlich sind folgende Messgrössen verwendbar:
a) Temperaturerhöhung ΔT °K bei gegebenem Heizstrom und Heizzeit: Dieses Prinzip ist vorbekannt, aber wegen der geringen Sensitivität weniger geeignet.
b) Die Aufheizrate in °K/sec beim Heizen bzw. die Abkühlrate (°K/sec) nach Abschalten der Heizung: Diese Werte sind über die Zeit nicht konstant und sind auch gegenüber Feuchteschwankungen nicht sehr empfindlich. c) Die notwendige Heizleistung (Ws) um eine bestimmte Temperaturerhöhung zu erreichen: Um mit diesem Prinzip eine hohe Sensitivität zu erzielen, muss die Heizleistung recht hoch angesetzt werden, was den Energieverbrauch hochtreibt. d) Die Zeit (sec) um eine bestimmte Temperatur wieder anzunehmen: Da nach dem Abschalten der Heizung die Temperatur exponentielle abklingt, kann man durch eine entsprechende Wahl einer Schwelltemperatur eine hohe Spreizung in diesem Messbereich und entsprechend eine hohe Sensitivität der Messung erreichen. In Versuchen hat sich daher diese Methode der Messung der Abkühlzeit als besonders vorteilhaft erwiesen. Durch eine entsprechende Wahl des Schwellwertes kann man sogar erreichen, dass der zur Steuerung der Bodenfeuchte relevante Feuchtebereich speziell gespreizt wird, sodass die Sensitivität maximiert wird. Ausserdem wird der Energiebedarf aufgrund eines nur kurzen Heizpulses minimiert. Bedingt durch die Messung von Temperaturdifferenzen, spielt die Höhe der Umgebungstemperatur keine Rolle.

Für die Optimierung der Sensitivität, sind folgende Kriterien für eine Messspitze vorteilhaft:
- Der eigentliche Sensorbereich sollte möglichst klein ausgeführt sein, da damit die zur Aufheizung notwendige Energie minimiert wird, wobei durch ein mikrothermisches Wärmemanagements in der Sensorspitze eine Zwischenspeicherung der Wärmeenergie in der Spitze erreicht werden kann.
- Die elektrische Verbindung zu einem Heiz- wie auch einem Messwiderstand sollten durch möglichst dünne elektrische Verbindungsleiter ausgeführt sein, damit möglichst wenig Wärmenergie durch Kabel abfliessen kann.
- Das vorderste Ende der Messspitze sollte thermisch gegenüber der übrigen Messspitze thermisch gut isoliert sein, da ansonsten eine zu grosse thermische Masse aufgeheizt und das Messergebnis entsprechend verfälscht wird, d.h es sollte eine minimale Wärmeleitung zur Halterung des eigentlichen Messfühlers und zu den Kabeln vorhanden sein.
- Die Messspitze ist mit einem standardisierten Interface versehen an welches die Wärmeenergie abgegeben wird. Das Interface ist zwischen dem Sensor und der Umgebung angeordnet, sodass ein Einfluss verschiedener umgebender Feststoffinedien, z. B. Bodenarten, wie Lehm, Sand, Torf etc. eliminiert oder zumindest stark verringert wird. Ein solches Interface, welches als auswechselbare Umhüllung an einem Sensor angebracht sein kann, ist bevorzugt aus einem mechanisch elastisch deformierbaren, saugfähigen Material, wie Filz, porösen Kunststoff, offenporiger Polyurethanschaum etc., welche eine hydrophile Oberfläche aufweisen, gefertigt, ist bevorzugt homogen in Bezug auf das Material und die Dichte, mit einer möglichst geringen thermische Leitfähigkeit.
- Die Messspitze ist in der Regel einer korrosiven Umgebung ausgesetzt. Sie bzw. der gesamte Sensorstab sollte daher aus einem Material bestehen, das entweder selbst einen hinlänglich guten Korrosionsschutz aufweist, oder aber beispielsweise mittels einer aufgebrachten Schutzschicht korrosionsfest gemacht werden.
- Ein Sensorstab mit eingebrachtem Sensor sollte auch einen relativ kleinen Durchmesser aufweisen, um das Einstechen des Stabes in die Erde oder andere Substrate zu erleichtern. Anderseits sollte er eine hinreichend hohe Festigkeit aufweisen, damit er beim Einstechen nicht knicken kann. Ausserdem ist es vorteilhaft, am Schaft des Messstabes eine Markierung anzubringen, die es gestattet, die Einstechtiefe abzulesen.
- Aluminium ist als Material für die Spitzenumhüllung ein sehr guter Kompromiss. Es weist eine relativ gute thermische Leitfähigkeit bei relativ niedriger Wärmekapazität auf, ist weitgehend korrosionsfest, hat insbesondere eine hydrophile Oberfläche und ist sehr leicht zu bearbeiten. Das Material ist auch sehr billig. Andere Materialien, wie z. B. rostfreier Stahl, Messing, Keramik etc. sind selbstverständlich auch möglich.

Wie bereits eingangs bei der Beschreibung des Messprinzips erwähnt, kann ein erforderliches Heizelement als Heizwiderstand getrennt von einem Messwiderstand ausgebildet sein, oder aber ein Messwiderstand wird gleichzeitig als Heizwiderstand eingesetzt. Im ersten Falle ist Ansteuerung und Messwerterfassung einfacher, im zweiten Falle ist kein zusätzlicher Heizwiderstand mit entsprechenden Zuleitungen erforderlich. Werden getrennte Widerstände verwendet, so kann als Messwiderstand ein so genannter Heissleiter eingesetzt werden, der eine sehr hohe Messgenauigkeit gewährleistet.

Es hat sich gezeigt, dass der thermischen Kopplung zwischen Sensor und umgebendem Medium, insbesondere bei Sensoren, die in ein Feststoffinedium eingebracht werden, eine wichtige Bedeutung zukommt. Es ist meist kein reproduzierbares Messergebnis erzielbar, wenn eine Sensorspitze unbedeckt in ein Substrat gesteckt wird, da die Grenzfläche, sowohl was die Grösse als auch die Beschaffenheit angeht, oft nicht definiert ist. Eine entsprechende Umhüllung des Sensors in der Form eines geeigneten Interface ist daher äusserst vorteilhaft.

Keramik mit einer gewissen Porosität ist dazu prinzipiell zwar geeignet, weist aber entscheidende Nachteile auf. Einerseits ist die Keramik hart und daher kann sie sich nicht einer unregelmässig geformten Umgebung, z.B. Erde, anpassen. Dies führt zu Luftspalten und einer nicht definierten Grösse der Grenzfläche. Andererseits weisen Keramikmaterialien (0.15 - 0.2W/m°K) eine gegenüber Luft (0.024 W/m°K) ca. 5 bis 10-fach grössere Wärmeleitung auf. Dies führt dazu, dass umgebendes Material, insbesondere Erde, auch im trockenen Zustand, thermisch schlecht vom Sensor entkoppelt ist. Der Sensor "spürt" die Erde auch wenn sie trocken ist. Dadurch wird die Sensitivität des Sensors negativ beeinflusst. Eine Schichtdicke zwischen Sensor und Erde müsste entsprechend gross gewählt werden, wobei Schichtdicken im Bereich von 5 bis 10 mm üblich sind. Dickere Schichten können Bereiche, die noch nicht mit Wasser gefüllt sind einschliessen, sodass aus diesen Bereichen die Luft nicht mehr entweichen kann. Solche Inhomogenitäten verfälschen die Messung. Eine poröse Keramik kann zudem nur ca. 30% Wasser aufnehmen. Dies ergibt ein maximales Verhältnis der Werte der Leitfähigkeit von Wasser und Keramik von nur 1.6 (Wasser und Erde hat ein Verhältnis von ca. 2, also noch höher). Zudem ist die Porenverteilung in der Keramik gegenüber der Porenverteilung in der Erde meist sehr verschieden. Die Erde hat je nach Art eine sehr unterschiedliche Porengrössen-Verteilung. Neben sehr kleinen Poren, z.B. in lehmigen Anteilen gibt es auch sehr grosse Poren in sandigen Substraten oder Torf. Eine poröse Keramik weist in der Regel nur sehr feine Poren auf, wodurch sie sich schnell mit Wasser voll saugt und sich auch bei kleinen Wasseranteilen im Substrat schnell sättigt. Sie ist daher praktisch immer mit Wasser gesättigt, auch wenn die Erde bereits recht trocken ist. Zudem gibt Keramik Wasser nicht mehr in dem Masse ab, die der effektiven Feuchte des Substrates entsprechen würde. Daher eignet sich eine poröse Keramik als Interface eigentlich nur für sehr trockne Bereiche.

Wegen der genannten Nachteile der Keramik wurde auch der Versuch unternommen, diese durch eine Standarderde zu ersetzen wie in DE 2536 777 erwähnt. Diese wird durch ein Netz am den Sensor gehalten, was wenig praktikabel ist. Trotz einer Standarderde ist die Berührungsfläche zwischen Sensor und Erde nicht gut definiert und verändert sich durch Wasser, das beim Giessen eingebracht wird. Die Erde wird ausgeschwemmt und verändert ihre thermischen Eigenschaften. Ausserdem sind die Probleme mit der geringen Sensitivität der Messung zu beachten, da immer das Gemenge Erde-Wasser gemessen wird und der Einfluss der Erde nicht eliminiert werden kann.

Umgebende Medien, wie Pflanzensubstrate, sind sehr heterogene Gemenge von feinporigen Anteilen, wie Lehm etc., Anteilen mit mittelgrossen und grossen Luftporen, wie Torf, Kompost oder auch Sand, sowie kleine oder auch grössere Steine, Erdklumpen etc. Zudem sind organischen Stoffe wie Pflanzenteile, Huminsäure etc. enthalten. Die thermischen Eigenschaften dieser Gemenge variieren sehr stark je nach Zusammensetzung, aber auch Füllgewicht, d.h. Dichte. So besitzt ein lose-geschüttetes Pflanzsubstrat weniger als die Hälfte der Wärmeleitfähigkeit im Vergleich zu einem zusammengepackten Substrat. Die Wärmeleitfähigkeit von trockenen Substraten variiert zwischen 0,3 und 2 W/m°K. Wasser besitzt eine Wärmeleitfähigkeit von 0.6 W/m°K, d.h. eine Differenzierung zwischen Substrat und Wasser ist nicht eindeutig und daher ist eine thermische Messung, wie beispielsweise in DE 4340775 beschrieben, schon allein aus diesem Grunde nicht aussagefähig. Mischt man trockenes Erdreich mit Wasser von 0 - 100% Wasseranteil so variiert die Wärmleitfähigkeit zwischen 0.3 und 0.6 W/m°K bzw. 2 bis 0.6 W/m°K. In der Praxis kann jedoch maximal nur ca. 50% Wasser von der Erde gebunden werden. Die Wärmekapazität von Erdreich liegt im Bereich von 1 - 3 Ws/g°K. Der Wert für Wasser ist 4.18 Ws/g°K und daher wesentlich höher. Eine Differenzierung über den Unterschied der Wärmekapazitätswerte ist aber praktisch nicht möglich, da die Wärmeleitung das Geschehen dominiert. Der Wärmefluss vom Sensor in das Erdreich wird bestimmt durch die bestehende Temperaturdifferenz, den Wärmeübergangswiderstand an der Grenzfläche, die Wärmeleitfähigkeit des Erdreichs, sowie die effektive Fläche durch die die Wärme strömen kann. Diese Erdeigenschaften sollten somit bei einer Feuchtemessung in Erden berücksichtigt werden um zuverlässige Messresultate zu erlagen. Dies wird unter anderem durch die Verwendung eines geeigneten Interfaces zwischen Sensor und Umgebung in der Form eines optimierten Materials erreicht.

Eine Umhüllung bzw. ein Interface sollte Feuchtigkeit vom Umgebungsmedium aufsaugen und wieder an dieses abgeben, vorzugsweise sollte dieser Vorgang jenem der Erde weitgehend entsprechen, d.h. Wasseraufnahme und -abgabe sollte analog und zeitgleich zur Erde erfolgen. Zudem sollte es mechanisch elastisch deformierbar sein, so dass es sich an eine nicht klar definierte Oberfläche eines Mediums, insbesondere eines heterogenen Stoffgemisches, anpasst und beispielsweise Eindrücke von Steinen oder Zwischenräume, von Erde, Schüttgut allgemein etc., ausgleicht. Die Umhüllung gleicht dadurch auch eine gewisse Volumenänderung des Mediums, bspw. durch Austrocknung oder Aufquellen, aus. Das für eine Umhüllung verwendete Material ist dazu offenporig mit vorzugsweise denselben Porenöffnungen wie ein umgebendes Feststoffmedium und vorzugsweise leicht mit Wasser benetzbar (hydrophil). Auch eine geringe Wärmeleitfähigkeit und Wärmekapazität der Umhüllung ist vorteilhaft, da ein Medium dadurch von einem Sensor -in trockenem Zustand- thermisch isoliert wird. Insbesondere Materialien aus Fasern, wie bspw. Filz, Gaze, Vlies, Gestricke oder Gewebe sind besonders geeignet aufgrund ihrer geringen Dichte. Synthetische Materialien für Umhüllungen sind besonders vorteilhaft, da sie nicht oder nur wenig verrotten. Materialien aus natürlichen Fasern können ihre Konsistenz ändern, wenn sie austrocknen, sie schrumpfen und können hart werden. Ausserdem verrotten sie teilweise schnell. Materialien aus Kunstfasern sind hingegen sehr formstabil und verrotten langsam oder gar nicht. Ein Interfacematerial sollte bevorzugterweise leicht bearbeitbar, leicht erhältlich und möglichst günstig sein.

Eine Umhüllung ist in einer bevorzugten Form auswechselbar an einem Sensor befestigt und kann bei Bedarf, z. B bei Alterungserscheinungen des Materials, wie Verkalkung oder Verrottung, aber auch zum Anpassen an andere Umgebungsmedien, ausgewechselt werden.

Filz hat sich als Umhüllung des Sensors in Versuchen als ausgezeichnetes Material herausgestellt. Es passt sich dank seiner Weichheit einer unregelmässigen Grenzfläche optimal an, ist sehr gut saugfähig und hat im trockenen Zustand einen sehr geringen Wänneleitwert, d. h. eine umgebende Erde wird thermisch sehr gut vom Sensor isoliert. Filzschichten weisen eine sehr kleine Wärmeleitfähigkeit von 0.03 W/m°K auf, was nur wenig über dem Wert von 0.024 W/m°K von Luft liegt und stellen demnach einen exzellenten Wärmeisolator dar. Im nassen Zustand hingegen, ist wegen der geringen Dichte des Filzes das absorbierte Wasser dominant. Zudem sind Filze sehr günstige Materialien, welche auch in dicken Schichten erhältlich sind. Filze, und allgemein Materialien aus Fasern, insbesondere vliesartige Materialien, haben den weiteren Vorteil, dass die Dichte angepasst werden kann. Dadurch kann das Saugverhalten an das von Erdsubstraten angepasst werden. Betrachtet man Wasser (0.6 W/m°K) im Verhältnis zu Filz (0.03 W/m°K), so ergibt sich ein Werteverhältnis von 20, d.h. man hat mit einem Filzinterface gegenüber der Erde eine 10-fache Sensitivität und gegenüber Keramik sogar eine noch höhere (ca. 12-fach). Ein solcher Filz oder anderes entsprechendes Interfacematerial verhält sich bezüglich thermischen Eingenschaften in trockenem Zustand im wesentlichen wie Luft und in nassem Zustand im wesentlichen wie Wasser.

In vielen Versuchsreihen haben sich Filze aus synthetischen Fasern als optimal herausgestellt, da diese gegenüber Pilzen sehr beständig sein können und demnach nicht oder nur sehr langsam verrotten. Bestimmte Fasern zeigen auch die Eigenschaft, Wasser rasch von der Umgebung aufzusaugen, aber auch wieder gemäss der Austrocknung der Umgebung an diese abzugeben, ohne dass ein signifikanter Unterschied im Wassergehalt zwischen Filz und Erde feststellbar wäre. Als besonders vorteilhaft hat sich die Verwendung von Filzen aus Aramidfasern herausgestellt. Sie vereinen in sich alle oben genannten Vorteile.

Der Sensor kann nicht nur zur Bestimmung der Feuchtigkeit von Pflanzenerde, sondern beispielsweise auch zur Überwachung der Feuchtigkeit von Mauerwerk, zur genauen Bestimmung des Aushärtegrades von Beton etc. eingesetzt werden. Weitere Einsatzmöglichkeiten liegen im Bereich der Überwachung von Bettnässern, im Bereich der Lebensmittel-Technologien, Messung des Reifegrades von Melonen usw.. Der Sensor ist aufgrund seiner Handlichkeit, preiswerten Ausführung, sowie seiner hohen Sensibilität auch in anderen Anwendungsbereichen vorteilhaft, z. B. in welchen keine inhomogenes umgebendes Medium vorhanden ist, beispielsweise bei Taupunktbestimmungen bzw. Taupunktüberwachungen. Sobald der Taupunkt erreicht wird, scheidet sich Wasser auf der Messspitze ab, was sich in einer Änderung der Abkühlkurve manifestiert. Der Taupunkt wird heute üblicherweise durch ein optisches Messsystem mit einem Spiegel gemessen oder mit Sensoren auf der Basis von elektronischen Feuchtesensoren, die eine über eine Feuchte absorbierende dielektrische Schicht den Taupunkt nachweisen. Beide Sensortypen sind sehr teuer, unterliegen Verschmutzung bzw. Korrosion und sind gerade für einfachere Anwendungen wirtschaftlich nicht tragbar. Der oben beschriebene erfindungsgemässe Sensor kann sehr kostengünstig hergestellt und zudem auch so verkapselt werden, dass keinerlei Korrosion oder Degradation auftritt. Für Taupunktsmessungen wird der Sensor vorteilhafterweise flächig ausgeführt. Der Heizwiderstand wird zusammen mit dem Messwiderstand (Kaltleiter) auf einem Metallblättchen thermisch kontaktiert und verkapselt. Der Sensor befindet sich zwischen den Messungen auf Umgebungstemperatur. Wird bei Erhöhung der Luftfeuchtigkeit oder Absinken der Temperatur das Metallblättchen betaut, so zeigt sich dies in einer Temperaturerhöhung, da Kondensationswärme frei wird. Bereits diese Temperaturerhöhung kann zum Nachweis der Betauung herangezogen werden, wobei durch eine Brückenschaltung mit einem vor Betauung geschützten Messwiderstand die Nachweisgenauigkeit wesentlich erhöht werden kann. Zudem kann der Sensor um wenige Grade aufgeheizt und analog zu den Feuchtemessungen der Temperaturverlauf der Abkühlkurve ausgewertet werden.

Das erfindungsgemässe Prinzip ist auch als Windsensor und Regensensor verwendbar. Wird der Sensor flächig ausgeführt und senkrecht zur normalen Regenrichtung plaziert, so kann die Oberfläche durch Regen benetzt werden. Dies kann sofort an der Messkurve nachgewiesen werden. Die Umgebungstemperatur spielt dabei keine Rolle, da eine Relativmessung durchgeführt wird. Um bereits bei den ersten Regentropfen einen sicheren Nachweis für den Regen zu erhalten, umfasst die Sensorfläche einen bevorzugten Bereich von ca. 10 cm2. In einer solchen Fläche können mehrere Sensoren untergebracht werden, die gemeinschaftlich oder einzeln ausgelesen werden können.

Grundsätzlich ist der Sensor immer dann einsetzbar, wenn eine dynamische Temperaturmessung mit aktiver Aufheizung des Sensors angebracht erscheint.

Die technischen Vorteile der Erfindung liegen auf der Hand:
- Eine hermetische Verkapselung des Sensors erlaubt den Einsatz auch unter erschwerten Umweltbedingungen. Eine Korrosion des Sensors kann verhindert und somit die Lebensdauer extrem verlängert werden.
- Die Verwendung eines standardisierten Interfaces, wie bspw. Filz, zwischen Sensor umgebendem Feststoffmedium, wie bspw. Erde, ist nicht nur eine technisch sehr einfache und auch billige Lösung, sie ergibt auch eine ausgezeichnete Sensorperformace, aufgrund der thermischen Trennung von Sensor und umgebendem Medium.
- In einer bevorzugten Ausführungsform enthält der Sensor keine Teile, die degradieren können, bzw. die routinemässig kontrolliert und allenfalls ausgewechselt werden müssen.
- Das Sensorsignal (Widerstandsänderung) ist sehr einfach zu messen und über einen Mikrocontroller auszuwerten.
- Da eine relative Messung durchgeführt wird, spielt die Umgebungstemperatur keine Rolle. Die definierte Heizleistung erhöht in einer bevorzugten Ausführungsform die Sensortemperatur praktisch immer um denselben Wert, unabhängig von der herrschenden Umgebungstemperatur.
- Der Sensor kann im Substrat in jeder beliebigen Tiefe plaziert werden, d.h. man kann die Feuchte direkt im Wurzelbereich von Pflanzen bestimmen und steuern.
- Die Heizleistung beträgt typischerweise 50 - 500 mW, die über eine typische Heizzeit von 5 - 30 Sekunden erbracht werden muss. Bei Messungen die üblicherweise alle Stunden durchgeführt werden, bedeutet dies einen sehr kleinen, mittleren Energiebedarf.
- Insbesondere die wirtschaftlichen Vorteile sind bedeutend. Die Herstellkosten in Seriequantitäten liegen im Bereich von 1- 2 Euro pro Stück, was gegenüber den heute gebräuchlichen Sensoren extrem niedrig ist. Dies bedeutet auch, dass bei grösseren zu überwachenden Bodenflächen zu gleichen Kosten wesentlich mehr Sensoren eingesetzt werden können.

Der hier dargestellte Sensor kann in unterschiedlicher Art und Weise angewendet werden. Zur Kontrolle bspw. der Bodenfeuchte ohne Rückkopplung wird der Sensor beispielsweise mit einer elektronischen Auswerteeinheit verbunden. Damit kann ein Gerät hergestellt werden, das erlaubt die Feuchte von Feststoffmedien, wie bspw. Erde in Blumentöpfen, Pflanztöpfen etc. zu kontrollieren, so dass manuell immer die richtige Bodenfeuchtigkeit eingestellt werden kann, z. B. für eine Pflanze durch manuelles Giessen.

Eine Anzeigeeinheit kann auf verschiedenste Art und Weise ausgeführt werden. Sie kann als separates Gerät mittels Kabel mit dem Messstab verbunden sein, oder aber Messstab und Elektronik werden als eine Einheit ausgeführt. Vorteilhafterweise wird die Stromversorgung durch integrierte Batterien bewerkstelligt. Nimmt die Messung nur einige Sekunden in Anspruch, ist der Stromverbrauch sehr niedrig.

Da verschiedene Pflanzen unterschiedliche Feuchtigkeit im Wurzelbereich verlangen, können durch einen integrierten Wahlschalter mehrere Pflanzengruppen gewählt werden: z. B. Trockenpflanzen, Normale, Feuchtpflanzen. Diese Einstellung verschiebt elektronisch den optimalen Feuchtebereich am Anzeigeinstrument. Eine eigentliche Anzeige besteht vorzugsweise aus einem LCD-Display, das auch andere Symbole beinhalten kann. Z. B. kann bei einem Nachweis von Staunässe bzw. von Trockenheit ein Alarmsymbol, z. B. verbunden mit einem Peep-Ton oder einem roten Alarmlicht ausgelöst werden. Zudem können Anzeigen über den Batterieladezustand integriert sein.

Zur Kontrolle der Bodenfeuchte mit Rückkopplung: Solche Systeme sind im Handel erhältlich, weisen aber Nachteile auf, die primär mit den verwendeten, nicht zuverlässig arbeitenden Sensoren zusammenhängen. Zudem sind sie entweder von einem lokal verfügbaren Stromnetz mit einer entsprechend spritzwasserdichten Zuleitung und/oder von einem Wasseranschluss abhängig.

Der oben beschriebene Sensor mit angeschlossener Auswerteelektronik gestattet die oben genannten Nachteile zu vermeiden und kann einfach und sehr kostengünstig zu einem Regelkreis zur Konstanthaltung der Bodenfeuchtigkeit ausgebaut werden. Dazu werden im wesentlichen folgende zusätzliche Komponenten benötigt:
- Eine elektronische Schaltung, die bei Unterschreiten einer zu definierenden Feuchtigkeit während einer durch die Elektronik zu bestimmende Zeitdauer eine Aktion auslöst, bspw. ein Ventil öffnet oder eine Pumpe anschaltet.
- Ein Wasserversorgungssystem, das entweder über einen Anschluss an das öffentliche Wassernetz besteht, oder über eine unabhängige Pumpe vorzugsweise mit Rückschlagventil, die aus einem Wasserreservoir Wasser beziehen kann.
- Ein Wasserverteilsystem in Form von Schläuchen, das Wasser an bestimmten Stellen dem Boden zuführt. Dieser Verteilschlauch kann sich auch in mehrere Äste verzweigen. Dadurch können mit einem System mehrere Pflanzkübel versorgt werden. Zudem kann der Sensorstab gleichzeitig auch als Zuführungsleitung verwendet werden.

Als besonders vorteilhafte Anordnung hat sich ein System erwiesen, das einerseits vom öffentlichen Stromnetz unabhängig ist und andererseits - wenn möglich - auch gesammeltes Regenwasser zur Bewässerung nutzen kann. Ein Beispiel eines solchen Systems wird nachfolgend beschrieben. Es besteht aus dem oben beschriebenen Sensor mit Auswerteelektronik. Diese ist in einem Gehäuse eingebaut, das neben der Elektronik auch eine Tauchpumpe enthält. Die Stromversorgung wird durch Batterien, eine Solarzelle, die über eine Ladeelektronik mit einem Akku verbunden ist, oder über das Stromnetz gewährleistet.

Das System beinhaltet zusätzlich einen Wahlschalter, über den unterschiedliche Pflanzenarten mit unterschiedlichem Wasserbedarf, eingestellt werden können. Das System ermittelt über den Sensor die aktuelle Feuchte im Wurzelbereich einer Pflanze, berechnet daraus unter Berücksichtigung des Wasserbedarfes der Pflanze eine zu liefernde Wassermenge d.h. eine Einschaltdauer für die Pumpe, die über die berechnete Zeit über das Verteilsystem Wasser in den Wurzelbereich der Pflanze zuführt. Dadurch wird die Feuchte erhöht. Der Sensor ermittelt in bestimmten Zeitintervallen, z.B. alle Stunde oder alle paar Stunden, die Feuchte neu und die Auswerteelektronik regelt über einen Regelalgorithmus die Feuchte auf ein für die Pflanze optimales Niveau ein.

Im Falle von Wasserzuflüssen durch Regen wird die Feuchte ohne Fremdeinwirkung erhöht. Die Regelanlage wird dann über einen bestimmten Zeitraum kein zusätzliches Wasser einbringen, so lange bis die optimale Feuchte unterschritten wird. Bei einem kurzzeitigen Regenguss mag das Wasser nicht bis in den Wurzelbereich einsickern, sondern verdunstet rasch wieder aus dem Oberflächenbereich. Die Regelung wird dadurch nicht gestört.

Bedingt durch die äusserst niedrigen Herstellkosten des Sensors ist es auch denkbar, dass mehrere Sensoren an verschiedenen Stellen Messungen durchführen, und so über eine Mittelung zu noch exakteren Werten gelangen oder um so flächenselektiv z. B. zu bewässern.

Eine Elektronik kann auch zusätzlich verschiedene Alarmsituationen feststellen und anzeigen, beispielsweise:

"Kein Wasser vorhanden": Diese Information kann aus der Leistungsaufnahme der Pumpe erhalten werden; "Akku leer"; "Frostgefahr": Die Temperatur des Substrates wird als Starttemperatur eines Messzyklus ohnehin bestimmt. Unterschreitet diese eine kritische Temperatur und der Boden droht zu frieren, wird Alarm ausgelöst. Dieser Alarm zeigt auch an, dass das System entleert werden muss, um Schäden durch Eisbildung zu verhindern. Des weiteren kann ein "Manuell-On" - Schalter vorgesehen werden, der dazu dient, die Pumpe über einen gewissen Zeitraum eingeschaltet zu lassen, um z.B. ein Wasservorratsgefäss leer zu pumpen. Die Integration eines Lichtsensors, z.B. einer einfachen Photodiode, erlaubt die automatische Feststellung von Tag und Nacht. Diese Information kann dazu verwendet werden, um die Bewässerung während der Tag- oder Nachtzeit zusätzlich zu beeinflussen.

Wird ein Wasservorratsbehälter eingesetzt, so kann man diesen mit zusätzlichen Funktionen ausstatten:
- Automatische Nachfüllung aus dem Wassernetz durch einen Schwimmschalter, analog der Wasserspülung im WC.
- Einbau eines Filtersystems, das das von der Pumpe zu fördernde Wasser hinreichend gut filtert, sodass keine Ablagerungen im Pumpsystem stattfinden können.
- Einbau eines Dünger-Vorratsbehälters, in dem Depot-Düngertabletten eingebracht werden können. Dieser gesonderte Bereich kann über kleine, allenfalls verstellbare Löcher mit dem Hauptvolumen verbunden sein. Die Dünger-Tabletten lösen sich im kleinen Wasservolumen langsamer auf, durch die Löcher kann aber mit Dünger angereichertes Wasser in den Hauptbehälter einsickern. Dadurch kann ein sehr gleichmässiger Düngereintrag erreicht werden.
- Einen durch ein Filter geschützten Einfüllstutzen, der zum manuellen Nachfüllen dient, oder aber mit einer Regenwasser-Sammelanlage verbunden ist.

Durch die oben aufgeführten Elemente resultiert einerseits eine extrem einfache Montage und Bedienung bei gleichzeitig sehr hoher Zuverlässigkeit und sehr niedrigen Gestehungskosten.

Die erfindungsgemässen Charakteristika werden anhand der folgenden beispielhaften Figuren näher dargestellt, dabei zeigt
- Fig.1: einen typischen Kurvenverlauf der Temperatur bei Auswertung der Aufheizphase
- Fig. 2: einen typischen Kurvenverlauf der Temperatur bei Auswertung der Abkühlphase
- Fig. 3: einen schematisch dargestellten Sensorstab
- Fig. 4: eine Schnittansicht durch die Spitze des Sensorstabes gemäss Fig. 3
- Fig. 5: ein gemessener Verlauf des Wassergehaltes von Blumenerde
- Fig. 6: eine schematische Anordnung mit wesentlichen Elementen eines Regelsystems zur automatischen Bewässerung
- Fig.7: einen Längsschnitt durch den vorderen Teil eines Sensorstabes
- Fig.: 8 der Längsschnitt gemäss Fig. 7 mit Interface
- Fig. 9: einen Querschnitt durch den Sensorstab gemäss Fig. 8

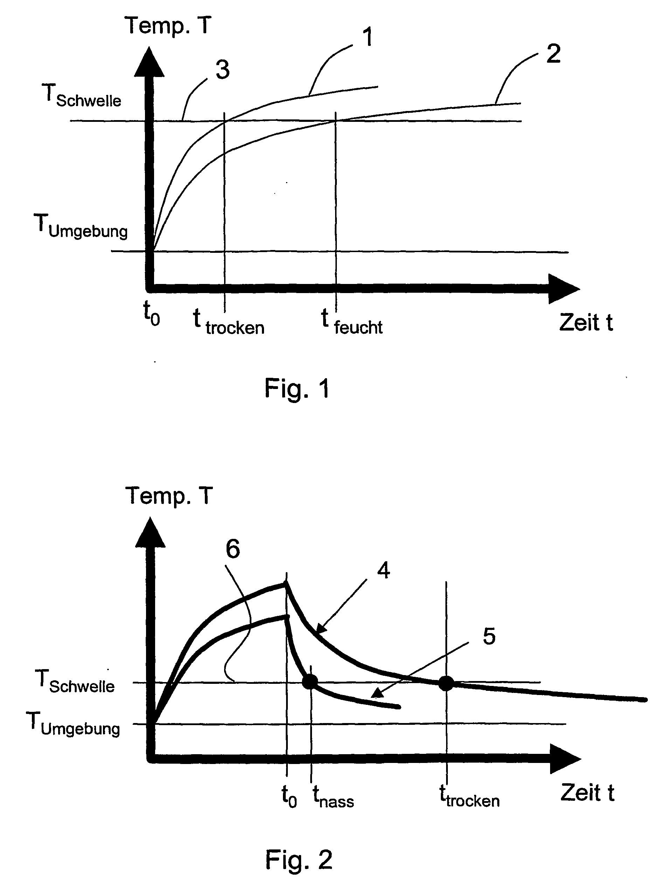

Figur 1 zeigt einen typischen Kurvenverlauf der Temperatur bei Auswertung der Aufheizphase bei hoher und niedriger Feuchte in einer Bodenprobe. Eine Kurve 1 zeigt den Temperaturverlauf für ein Substrat mit niedrigem Feuchtegehalt, eine weitere Kurve 2 den Temperaturverlauf für ein feuchtes Substrat. Einem Temperaturschwellwert 3 können so zwei Zeiten, t trocken bzw. t feucht, entsprechend den Kreuzungspunkten des Schwellwerts mit den beiden Kurven 1, 2 zugeordnet und bei Unterschreiten einer kritischen Zeit eine Aktion ausgelöst werden.

**Figur 2** zeigt einen typischen Kurvenverlauf der Temperatur mit Auswertung der Abkühlphase bei hoher und niedriger Feuchte in einer Bodenprobe. Eine Kurve 4 zeigt wiederum den Temperaturverlauf für ein Substrat mit niedrigem Feuchtegehalt, eine weitere Kurve 5 den Temperaturverlauf für ein feuchtes Substrat. Einem gegebenen Temperaturschwellwert 6 werden durch die Kreuzungspunkte des Schwellwertes mit dem absteigenden Ast der beiden Kurven, wiederum zwei Zeiten, t trocken bzw. t feucht, zugeordnet und bei Überschreiten einer kritischen Zeit eine Aktion ausgelöst. Der Temperaturschwellwert kann entweder für alle Feuchtegrade konstant sein, oder aber je nach Feuchtegrad verschieden gewählt werden.

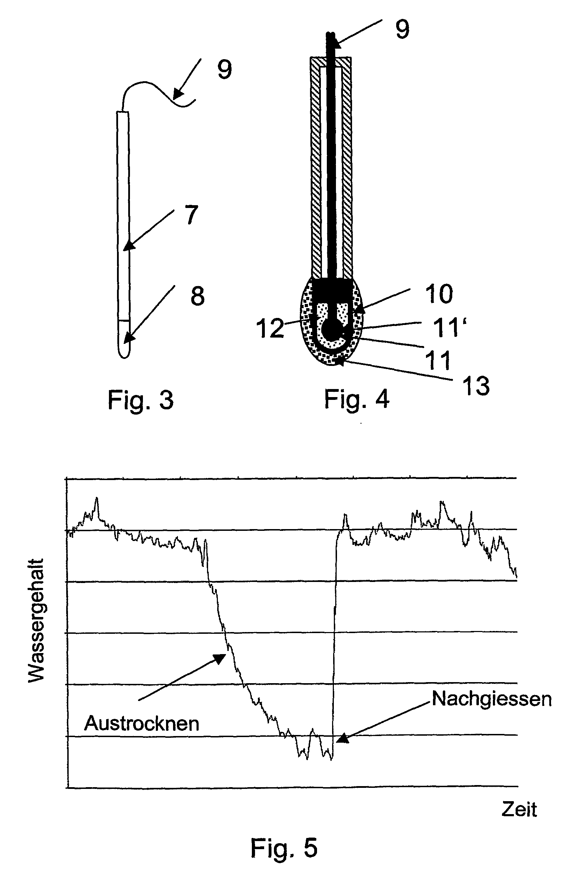

**Figur 3** zeigt einen Sensorstab 7 mit eigentlichem Sensor 8 an der Spitze des Sensorstabes. Der Sensorstab 7 besteht zumindest im Sensorbereich aus einem thermisch schlecht leitenden Material, wie Kunststoff, Keramik etc. Am Ende des Messstabes erkennt man ein Kabel 9, das alle Drahtverbindungen zur Heizung und zum Messwiderstand (in der Figur nicht gezeigt) umfasst.

**Figur 4** zeigt einen Schnitt durch die Spitze bzw. den vorderen Teil des Sensorstabes gemäss Fig. 3. Man erkennt die Schutzhülle 10, in der der Temperatursensor 11 und der Heizwiderstand 11' beispielsweise Mittels eines Klebers 12 eingeklebt sind. Das obere, hintere Ende des Sensorstabes ist nur schematisch dargestellt. Das Kabel 9 wird aus dem hohlen Sensorstab über eine Dichtungsmuffe in ein isoliertes und flexibles Kabel übergeführt. Die Spitze ist mit einem saugfähigen Überzug 13, z. B. einer Filzkappe, versehen. Es ist auch möglich den gegebenenfalls eingeklebten Temperatursensor und Messwiderstand, mit einem geeigneten Interface zu überziehen und dieses mit einer verstärkten Spitze bzw. Kappe zu versehen. Eine solche Spitze weist vorzugsweise nur wenige, dünne, über das Interface führende Stege auf und endet in einer vorzugsweise leicht zugespitzten massiveren Spitze. Eine solche Spitze erlaubt das Eindrücken des Sensorstabes in die Erde, ohne dass Interface oder Messwiderstand Schaden nehmen.

**Figur 5** zeigt den mit einer erfindungsgemässen Vorrichtung gemessenen Verlauf des Wassergehaltes über die Zeit einer Blumenerde in einem Blumentopf, bepflanzt mit einem Weihnachtsstern. Man erkennt die Abnahme des Wassergehaltes, bzw. das Austrocknen der Erde. Nach dem Nachgiessen nimmt der Wassergehalt sprunghaft wieder zu, was sich in einem steilen Anstieg der Messkurve bemerkbar macht.

Die Zeit zwischen zwei Messungen beträgt zwischen 0.5-5 Stunden, typisch sind Zeiten zwischen 1-2 Stunden.

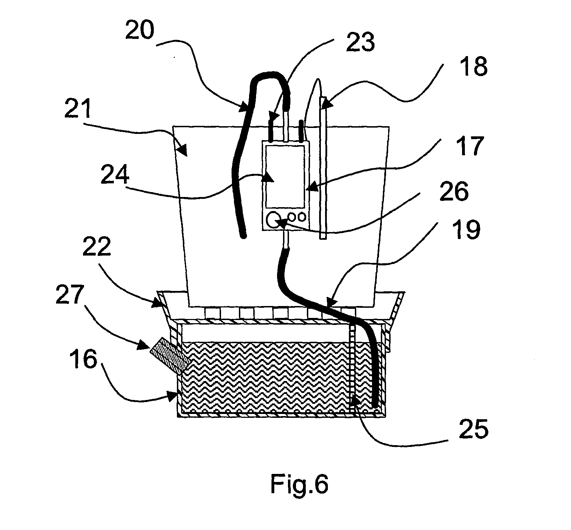

In dem in **Figur 6** gezeigten Regelsystem zur automatischen Bewässerung steht ein Pflanzkübel 21 auf einem Wasservorratsgefäss 16. Der Deckel des Gefässes 16 ist als Auffangwanne 22 für aus dem Pflanzkübel ablaufendes Wasser ausgebildet. Das Vorratsgefäss ist durch eine Filterwand 25 unterteilt, wobei Wasser durch einen Einfüllstutzen 27 in das Vorratsgefäss 16 einfliesst. Auf der dem Filter 25 gegenüberliegenden Seite des Gefässes wird es für die Bewässerung entnommen. Dadurch kann eine Verstopfung von Leitungen weitgehend verhindert werden, bzw. nur kleine Wasservolumina sind z.B. mit Dünger versetzbar. Am Pflanzkübel ist über eine einfache mechanische Befestigung 23, z. B. Bügel, ein Steuergerät 17 befestigt. Das Steuergerät enthält sowohl die Auswerteelektronik für das vom Sensor 18 gelieferte Signal, als auch eine Pumpe, die über eine Saugleitung 19 Wasser über eine Verteilleitung 20 in den Pflanzkübel 21 einleitet. Das Steuergerät bezieht seine Energie aus einer Solarzelle 24 und kann verschiedene Bedien- und Anzeigeelemente 26 enthalten.

Es versteht sich von selbst, dass das gesamte Regelsystem auch anders angeordnet sein kann: z.B. Wasservorratsgefäss 16 und Pflanzkübel 21 mit Auffangwanne 22 nebeneinander und Steuergerät 17 neben oder auf dem Pflanzkübel. Zudem kann die Verteilleitung 20 sich in mehrere Leitungen aufteilen, sodass gleichzeitig mehrere Pflanzen bedient werden können.

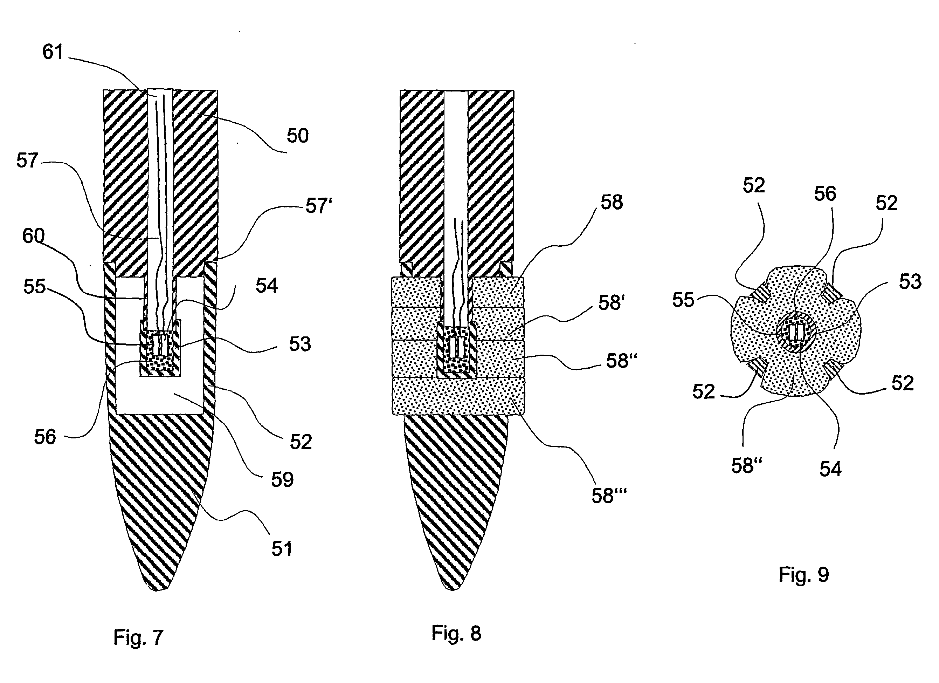

**Figur 7** zeigt einen Längschnitt durch einen vorderen Bereich einer weiteren Ausführungsform eines erfindungsgemässen Sensorstabs. An einem nicht vollständig gezeichneten Schaft 50 wird eine zugespitzte Kappe 51, vorzugsweise stoff - oder formschlüssig, befestigt, derart dass sie sich möglichst auch beim Herausziehen aus einem zusammengepressten Erdsubstrat nicht vom Schaft löst. Die Kappe 51 erlaubt ein einfaches Einstecken des Sensors bzw. Sensorstabs in ein Substrat. Schaft und Kappe sind vorzugsweise beide aus Kunststoff gefertigt. Die Kappe 51 besitzt im Sensorbereich eine laternenartige Struktur, d. h. ein von der Kappe gebildeter Hohlraum 59 weist mehrere, im Minimum zwei, bevorzugt jedoch auch drei oder vier, Ausschnitte auf, sodass die Kappe 51 nur mit zwei oder mehreren Stegen 52 mit dem Schaft verbunden ist. Dadurch entstehen Fenster, durch die der eigentliche Sensor zugänglich ist. Der Sensor weist eine metallische Abdeckhülse 53 auf, in der der Heizwiderstand 54 und der Messwiderstand 55, vorzugsweise Rücken an Rücken positioniert, mit einer Vergussmasse 56 eingossen sind. Möglichst dünne Drähte 57, 57' verbinden die Widerstände mit einer Ansteuervorrichtung.

Um möglichst wenig Wänneenergie vom Sensor in den Schaft 50 abzuleiten, weist der Schaft eine Verlängerung in Form eines dünnwandigen Rohres 60 auf, auf dem die Abdeckhülse 53 befestigt werden kann, beispielsweise aufgesteckt und mit der Vergussmasse 56 verklebt. Ein im Schaft vorhandenes Durchgangsloch 61 dient zur Zuführung der Drähte. Dadurch wird der Sensor auch auf Seiten des Schafts optimal von der Umgebung thermisch isoliert.

Die in den Sensor eingebrachte Wärmemenge nicht sofort an die Umgebung abgegeben, sondern grösstenteils im Sensor zwischengespeichert wird. Dazu ist die Befestigung der Metallhülse am weiterführenden Sensorteil als sehr dünnwandiges Kunststoffrohr ausgeführt ist. Dies verhindert weitgehend einen Abfluss der applizierten Wärmeenergie an den restlichen Teil der Sensorkonstruktion. Zudem werden die elektrischen Zuleitungen zum Heiz- und Messwiderstand als sehr dünne Kupferleitungen ausgeführt, die ebenfalls nur sehr wenig Wärmeenergie nach hinten in Richtung Sensorschaft wegleiten können. Auch ist die Sensorspitze aus diesem Grunde mit der Vergussmasse bzw. einem Kleber ausgegossen, der keinen direkten thermischen Kontakt zwischen Heizwiderstand 54 und umgebender Metallhülse erlaubt. Der Heizwiderstand gibt die Wärmeenergie an diese Vergussmasse ab, die sie anschliessend an die Metallhülse weitergibt. Zu diesem Zeitpunkt wird die Heizung abgeschaltet und der Messwiderstand misst nun die Temperaturabnahme durch die Weitergabe der Wärmeenergie an den mit Wasser getränkten Filz in der unmittelbaren Umgebung, wie in Figuren 8 und 9 gezeigt. Die Erde wird dabei - im Unterschied zu den meisten anderen Messverfahren - nicht erwärmt, sofern die Dicke des Filzes nur einige wenige mm beträgt, z. B. in einem Bereich von 2- 6 mm, vorzugsweise 3-5mm, z. B. 4.5mm. Die Dicke wird derart gewählt, dass ein Einfluss des umgebenden Mediums auf die Messung vernachlässigbar ist, also beispielsweise <10%, vorzugsweise ≤5% ist. Bei abnehmender Filzdicke nimmt der thermische Einfluss der Erde zu und der Sensor wird unsensibler und von der Art des umgebenden Mediums stärker beeinflusst.

Beipielhafte Wertebereiche von Dichten von Filzen sind 0.05g/cm³-0.3g/cm³, vorteilhafterweise 0.1-0.2g/cm³, z.B. 0.14g/cm³. Je nach Dichte und Beschaffenheit von umgebenden Medium kann die Dicke entsprechend variiert werden.

**Figur 8** zeigt einen Schnitt durch den vorderen Teil des Sensorstabes mit Sensor nach Einbringen eines Inferfaces. Dies ist ein saugfähiges, mechanisch leicht deformierbares Materials mit möglichst kleiner Wärmeleitfähigkeit, vorzugsweise Filz. Das Material ist in dieser Ausführungsform in der Form von vier aufeinander geschichteten Scheiben dargestellt, wobei drei dieser Scheiben 58, 58', 58" ein zentrales Loch aufweisen, in welchen der Sensor rundum seitlich eingebettet ist. Die Scheibe 58"', welche am nächsten zur vordersten Sensorspitze liegt, weist kein zentrales Loch auf Dadurch wird auch der Boden der metallischen Abdeckhülse mit Filz abgedeckt. Das Interface kann auch anders gestaltet sein, z.B. einstückig.

Aus Figur 8 und in **Figur 9** im Querschnitt durch den Stab auf der Höhe des Sensors gezeigt, ist ersichtlich, dass der Aussendurchmesser der saugfähigen Filzscheiben wenig grösser als der Aussendurchmesser des Schafts bzw. der Sensorstabsspitze ist, wodurch das Material zwischen den Stegen 52 aus den Fenstern teilweise herausgedrückt wird. Dies verbessert einerseits den Kontakt mit dem umgebenden Erdsubstrat, wodurch auch bei lockerer Erde die Feuchte gut aufgenommen wird, aber auch die thermische Separation zur Erde, da die Schichtdicke des saugfähigen Materials bei gegebenem Sensorstabdurchmesser um den Sensor herum noch erhöht wird. Im Bereich der Stege entsteht zudem ein Druck des saugfähigen Materials in Richtung des Sensors, was eine gute und zeitlich stabile thermische Kontaktierung zwischen Filz und Sensor gewährleistet. Die in Figur 9 dargestellte Situation weist aber noch einen weiteren wesentlichen Vorteil auf. In den teilweise komprimierten Filzscheiben werden lokal dichtere - im Bereich der Stege - und weniger dichte Zonen - im Bereich der Fenster - erzeugt, d.h. ein ganzes Spektrum an verschiedenen Porengrössen. Dadurch wird eine wesentlich bessere Angleichung der Porenverteilung an jene der Erdsubstrate erzielt und die Messung des Feuchtegehaltes wird wesentlich genauer.

Die gezeigte Sensorkonstruktion reflektiert in optimaler Art die Erfordernisse eines auch in realen Erdsubstraten funktionierenden, thermischen Messverfahrens. Sie weist eine möglich gute thermische Isolierung des Sensors von der Umgebung, einschliesslich Sensorschaft und Spitze auf Durch diese Massname und eine nur kurzzeitige Temperaturerhöhung um wenige Grad wird erreicht, dass nur die unmittelbare Nachbarschaft des Sensors für die Messung relevant ist Es kann erreicht werden, dass an der Grenzfläche Filz - Erde praktisch keinerlei Temperaturerhöhung mehr festgestellt werden kann. Zudem wird durch ein geeignetes Interface eine optimale Extraktion des Wassers von der Umgebung erreicht. Eine mechanisch anpassungsfahige Grenzfläche, die auch bei lockerer Erde oder grobkörnigem Substrat, wie beispielsweise Blähton oder poröses Gestein, wie es für die Bepflanzung, insbesondere von Dachgärten, Verwendung findet, bildet eine zuverlässige Feuchtebrücke. Durch die Bildung eines Porenspektrums, das durch eine gezielte, lokale Komprimierung des saugfähigen Materials erreicht wird, ist Aufnahme und Abgabe des Wassers im Gleichschritt mit dem des umgebenden Substrates. Durch eine im wesentliche vollständige Trennung von Messfühler und umgebendem Medium fällt eine bei anderen Messverfahren notwendige Eichung zur Berücksichtigung der thermischen Eigenschaften des Mediums weg, was das Messverfahren einfacher und weniger Fehler anfällig macht.

Mit dem Verfahren ist es somit möglich, nicht das ErdeWassergemisch zu messen, sondern das Wasser von der Erde oder einem anderen, insbesondere auch heterogenen, Feststoffmedium zu extrahieren, in ein homogenes Interface zu überführen, thermisch zu isolieren und mit hoher Sensitivität die Abkühlkurve zu messen.

## Patentansprüche

1. Vorrichtung aufweisend mindestens einen Messfühler bestehend aus einem Temperatursensor (3) und ein Mittel (7) zum Erwärmen desselben, sowie eine Beschaltung bestehend aus einer Auswerteelektronik (15) und einem Steuergerät (17), die der Erwärmung des beheizbaren Temperatursensors und der Bestimmung des Feuchtegehalts eines den Messfühler umgebenden Mediums dient, **dadurch gekennzeichnet, dass** zwischen Messfühler und umgebendem Medium eine Zwischenschicht angeordnet ist, wobei der Messfühler von dieser Zwischenschicht aus einem saugfähigen, mechanisch deformierbaren und thermisch isolierenden Material (13) aus synthetischen Fasern und/oder aus Filz umgeben ist.

2. Vorrichtung nach Anspruch 1, wobei das Material (13) den Messfühler umhüllt.

3. Vorrichtung nach Anspruch 1 oder 2, wobei das Material (13) aus Aramidfasern

4. Vorrichtung nach einem der Ansprüche 1 - 3, wobei das Material (13) Feuchte aufsaugend und abgebend, sowie offenporig ist.

5. Vorrichtung nach einem der Ansprüche 1 - 4, wobei das Material (13) homogen, insbesondere homogen bezüglich seiner Dichte ist.

6. Vorrichtung nach einem der Ansprüche 1 - 4, wobei das Material (13) lokal komprimiert ist derart, dass ein Porengrössenspektrum vorhanden ist.

7. Vorrichtung nach einem der Ansprüche 1-6, wobei der Temperatursensor (3) und das Mittel (7) zum Erwärmen im wesentlichen vollständig in eine elektrisch isolierende Masse (12, 56) eingebettet sind, welche Masse (12, 56) von einer Hülse (53) aus thermisch leitfähigem Material umgeben ist und die Hülse in direktem mechanisch und thermischem Kontakt mit dem saugfähigen, mechanisch deformierbaren und thermisch isolierenden Material (13) aus synthetischen Fasern und/oder Filz ist.

8. Vorrichtung gemäss einem der Ansprüche 1-7, wobei die Dicke des saugfähigen, mechanisch deformierbaren und thermisch isolierenden Materials (13) aus synthetischen Fasern und/oder Filz in einem Bereich von 3-7mm liegt.

9. Vorrichtung gemäss einem der Ansprüche 1-8, wobei Vorrichtungsteile (3, 7, 7') in kontrollierter Weise über eine bestimmte Zeit aufheizbar und anschliessend selbstständig abkühlbar ausgestaltet sind und dass die Beschaltung (15, 17) so ausgelegt ist, dass nebst Beheizung, Zeitspannen zum Erreichen von Temperaturschwellwerten mess- und auswertbar sind.

10. Vorrichtung nach Anspruch 9, wobei bei Erreichen einer vorgegebenen Zeitspanne Aktionen auslösbar sind.

11. Vorrichtung nach einem der Ansprüche 1-10, mit Mittel zum Fördern eines Fluids, wobei diese Mittel durch Aktionen aus der Beschaltung (15, 17) betätigbar sind.

12. Verwendung der Vorrichtung nach einem der Ansprüche 1-11 als Feuchtesensor.

13. Verwendung der Vorrichtung nach einem der Ansprüche 1-12 zum automatischen Bewässern von Pflanzen.

## Claims

1. Device comprising at least one measurement probe consisting of a temperature sensor (3) and a means (7) for heating this, as well as a circuiting consisting of evaluation electronics (15) and a control device (17), which serves for heating the heatable temperature sensor and for determining the moisture content of a medium surrounding the measurement probe, **characterized in that** between the measurement probe and the surrounding medium an intermediate layer is arranged, wherein the measurement probe is surrounded by this intermediate layer made of an absorbent, mechanically deformable and thermally insulating material (13) of synthetic fibres and or of felt.

2. Device according to claim 1, wherein the material (13) encloses the measurement probe.

3. Device according to claim 1 or 2, wherein the material (13) consists of aramide fibre.

4. Device according to one of claims 1 to 3, wherein the material (13) absorbs and releases moisture, and is open-pored.

5. Device according to one of claims 1 to 4, wherein the material (13) is homogeneous, in particular homogeneous with regard to its density.

6. Device according to claim 1 to 4, wherein the material (13) is locally compacted in a manner such that a pore size spectrum is present.

7. Device according to one of the claims 1 to 6, wherein the temperature sensor (3) and the means (7) for heating are substantially completely embedded in an electrically insulating mass (12, 56), said mass (12, 56) being surrounded by a sleeve (53) of thermally conductive material, and the sleeve is in direct mechanical and thermal contact with the absorbing, mechanically deformable and thermally insulating material (13) made of synthetic fibres and/or felt.

8. Device according to one of the claims 1 to 7, wherein the thickness of the absorbing, mechanically deformable and thermally insulating material (13) made of synthetic fibres and/or felt is in a range of 3-7mm.

9. Device according to one of the claims 1 to 8, wherein device parts (3, 7, 7') are designed to be heatable over a certain time in a controlled manner, and to be subsequently coolable automatically, and wherein the circuiting (15, 17) is designed such that apart from the heating, time durations for reaching temperature thresholds may be measured and evaluated.

10. Device according to claim 9, wherein actions may be triggered on reaching a predefined time duration.

11. Device according to one of the claims 1 to 10, with means for conveying fluid, wherein these means may be actuated by way of actions from the circuiting (15, 17).

12. Use of the device according to one of the claims 1 to 11 as moisture sensor.

13. Use of the device according to one of the claims 1 to 12 for the automatic irrigation of plants.

## Revendications

1. Dispositif qui présente au moins une sonde de mesure constituée d'un détecteur de température (3) et d'un moyen (7) de chauffage de ce dernier, ainsi qu'un circuit constitué d'une électronique d'évaluation (15) et d'un appareil de commande (17) qui sert à chauffer le détecteur de température chauffable et à déterminer la teneur en humidité d'un fluide qui entoure la sonde de mesure,
**caractérisé en ce que**
une couche intermédiaire est disposée entre la sonde de mesure et le fluide environnant, la sonde de mesure étant entourée par cette couche intermédiaire en un matériau (13) en fibres synthétiques et/ou en feutre, absorbant, mécaniquement déformable et thermiquement isolant.

2. Dispositif selon la revendication 1, dans lequel le matériau (13) enveloppe la sonde de mesure.

3. Dispositif selon les revendications 1 ou 2, dans lequel le matériau (13) est constitué d'aramide.

4. Dispositif selon l'une des revendications 1 à 3, dans lequel le matériau (13) absorbe et libère l'humidité et est à porosité ouverte.

5. Dispositif selon l'une des revendications 1 à 4, dans lequel le matériau (13) est homogène, et en particulier présente une densité homogène.

6. Dispositif selon l'une des revendications 1 à 4, dans lequel le matériau (13) est comprimé localement de manière à présenter un spectre de tailles de pores.

7. Dispositif selon l'une des revendications 1 à 6, dans lequel la sonde de température (3) et le moyen (7) de chauffage sont incorporés essentiellement complètement dans une pâte électriquement isolante (12, 56), laquelle pâte (12, 56) est entourée par un fourreau (53) en matériau thermiquement conducteur, le fourreau étant en contact mécanique et thermique direct avec le matériau (13) en fibres synthétiques et/ou en feutre, absorbant, mécaniquement déformable et thermiquement isolant.

8. Dispositif selon l'une des revendications 1 à 7, dans lequel l'épaisseur du matériau (13) en fibres synthétiques et/ou en feutre, absorbant, mécaniquement déformable et thermiquement isolant est comprise dans la plage de 3 à 7 mm.

9. Dispositif selon l'une des revendications 1 à 8, dans lequel des parties (3, 7, 7') du dispositif sont configurées de manière à pouvoir être chauffées de manière contrôlée pendant un certain temps et ensuite à pouvoir se refroidir de manière autonome et en ce que les circuits (15, 16) sont conçus de telle sorte qu'en plus du chauffage, ils permettent de mesurer et d'évaluer la durée nécessaire pour atteindre une valeur de seuil de température.

10. Dispositif selon la revendication 9, dans lequel des actions peuvent être déclenchées lorsqu'une durée prédéterminée s'est écoulée.

11. Dispositif selon l'une des revendications 1 à 10, qui présente des moyens de transport d'un fluide, ces moyens pouvant être actionnés par des actions du circuit (15, 17).

12. Utilisation du dispositif selon l'une des revendications 1 à 11 comme détecteur d'humidité.

13. Utilisation du dispositif selon l'une des revendications 1 à 12 pour l'arrosage automatique de plantes.
